# EUROPEAN PATENT SPECIFICATION

(11) **EP 3 598 939 B1**
(45) Date of publication and mention of the grant of the patent: **07.06.2023**
(21) Application number: 19188558.1
(22) Date of filing: 26.07.2019
(51) Int. Cl.: A61B 5/0205, A61B 5/053, A61B 5/145

(54) **APPARATUS AND METHOD FOR MEASURING A BIOSIGNAL**
GERÄT UND VERFAHREN ZUR MESSUNG EINES BIOGNALS
APPAREIL ET PROCEDE DE MESURE D'UN BIOSIGNAL

(30) Priority: 27.07.2018 KR 20180088098
(43) Date of publication of application: 29.01.2020
(73) Proprietor: Samsung Electronics Co., Ltd., Gyeonggi-do 16677 (KR)
(72) Inventor: JUNG, Myoung Hoon, Gyeonggi-do (KR); EOM, Kun Sun, Gyeonggi-do (KR); MOON, Hyun Seok, Seoul (KR); SHIM, Jae Wook, Gyeonggi-do (KR); JANG, Hyeong Seok, Seoul (KR)
(74) Representative: Grünecker Patent- und Rechtsanwälte PartG mbB

(56) References cited:
- WO-A1-2010/044026
- US-A1- 2006 025 701
- US-A1- 2016 066 812
- US-A1- 2018 042 557
- US-B2- 7 865 236

## Description

### BACKGROUND

### 1. Field

The disclosure relates to an apparatus and method for measuring a biosignal.

### 2. Description of Related Art

A variety of medical devices are being developed to diagnose health conditions of patients. The importance of medical devices for measuring electrical biosignals of patients has been increasingly recognized in terms of convenience to patients and promptness of diagnosis results during health condition diagnosis. For example, US 2006/025701 and US 2016/066812 describe devices, which determine body composition from electrical biosignals.

The skin of the living tissues is composed of various layers, such as stratum corneum, epidermis, dermis, and the like, and each layer has different characteristics. In order to measure a desired biosignal, information of a desired layer among several layers may be determined. For example, in the case of an impedance spectroscopy based blood glucose sensor, impedance information of blood from the dermis may be determined. However, the impedance determined in such a manner is influenced by other living tissues of the skin and, thus, acquiring information regarding the other living tissues is also required. To this end, attempts have been made to use different types of sensors in an aggregated manner by constructing a composite sensor using the different sensors. For example, US 2018/042557 describes a composite sensor having biosignal electrodes arranged around a central photoplethysmography sensor.

### SUMMARY

This summary is provided to introduce a selection of concepts in a simplified form that are further described below in the Detailed Description. This summary is not intended to identify key features or essential features of the claimed subject matter, nor intended to be used as an aid in determining the scope of the claimed subject matter, which is defined by the appended claims.

The following description relates to an apparatus and method for measuring a biosignal.

According to an aspect of the disclosure, there is provided an apparatus for measuring a biosignal, including an optical sensor configured to emit light in a measurement region to an object of interest and receive an optical signal reflected from the object of interest, and a bioelectrical impedance sensor configured to measure a depth-specific bioelectrical impedance in the measurement region of the optical sensor.

According to the invention, the bioelectrical impedance sensor measures the depth-specific bioelectrical impedance by adjusting a spacing of electrodes, an area of an electrode, or a measurement frequency.

The bioelectrical impedance sensor may include a first electrode and a second electrode that are spaced a first distance apart from each other and are disposed symmetrically with respect to the optical sensor, a third electrode and a fourth electrode that are spaced a second distance apart from each other and are disposed symmetrically with respect to the optical sensor, and an impedance measurer configured to measure a first bioelectrical impedance at a first depth using the first electrode and the second electrode, and measure a second bioelectrical impedance at a second depth using the third electrode and the fourth electrode.

The first electrode and the second electrode may be interposed between the third electrode and the fourth electrode.

Respective centers of the first electrode, the second electrode, the third electrode, the fourth electrode, and the optical sensor may form a straight line.

The bioelectrical impedance sensor may include a mode controller configured to control an operation mode for measuring the depth-specific bioelectrical impedance.

The mode controller may connect the impedance measurer to the first electrode and the second electrode in a first operation mode; and connect the impedance measurer to the third electrode and the fourth electrode in a second operation mode.

The impedance measurer may include a current source configured to apply a first current to the object of interest via the first electrode and the second electrode in the first operation mode; and apply a second current to the object of interest via the third electrode and the fourth electrode in the second operation mode; and a voltmeter configured to measure a first voltage applied between the first electrode and the second electrode in the first operation mode; and measure a second voltage applied between the third electrode and the fourth electrode in the second operation mode.

The mode controller may disconnect the first electrode and the third electrode in a first operation mode; disconnect the second electrode and the fourth electrode in the first operation mode; connect the first electrode and the third electrode in a second operation mode; and connect the second electrode and the fourth electrode in the second operation mode.

The impedance measurer may include a current source configured to apply a first current to the object of interest via the first electrode and the second electrode in the first operation mode; and apply a second current to the object of interest via the third electrode that is connected to the first electrode and via the fourth electrode that is connected to the second electrode in the second operation mode; and a voltmeter configured to measure a first voltage applied between the first electrode and the second electrode in the first operation mode; and measure a second voltage applied between the third electrode that is connected to the first electrode and the fourth electrode that is connected to the second electrode in the second operation mode.

The bioelectrical impedance sensor may include a first electrode and a second electrode which are spaced a predetermined distance apart from each other and are disposed symmetrically with respect to the optical sensor; and an impedance measurer configured to measure a first bioelectrical impedance at a first depth using the first electrode and the second electrode; and measure a second bioelectrical impedance at a second depth using the first electrode and the second electrode.

The bioelectrical impedance sensor may include a mode controller configured to control an operation mode for measuring the depth-specific bioelectrical impedance.

The mode controller may set the measurement frequency to a first frequency in a first operation mode; and set the measurement frequency to a second frequency in a second operation mode.

The impedance measurer may include a current source configured to apply a first current of the first frequency to the object of interest via the first electrode and the second electrode in the first operation mode; and apply a second current of the second frequency to the object of interest via the first electrode and the second electrode in the second operation mode; and a voltmeter configured to measure a voltage applied between the first electrode and the second electrode.

According to the invention, the apparatus further includes a processor configured to: determine biometric information of the object of interest based on the received optical signal and the depth-specific bioelectrical impedance.

The biometric information may include a body fat mass, a fat-free mass, a muscle mass, a skeletal muscle mass, a basal metabolic rate, an intracellular water mass, an extracellular water mass, a body water mass, an inorganic mass, a visceral fat mass, a blood flow volume, a calorie intake, a hematocrit, or a blood glucose level.

According to an aspect of the disclosure, there is provided a method of measuring a biosignal which is performed by an apparatus for measuring the biosignal that includes an optical sensor, a first electrode and a second electrode that are spaced a first distance apart from each other and are disposed symmetrically with respect to the optical sensor, and a third electrode and a fourth electrode that are spaced a second distance apart from each other and are disposed symmetrically with respect to the optical sensor, the method may include measuring an optical signal by emitting light in a measurement region to an object of interest and receiving light reflected from the object of interest; and measuring a depth-specific bioelectrical impedance in the measurement region of the optical sensor using the first electrode, the second electrode, the third electrode, and the fourth electrode.

According to the invention, the measuring of the depth-specific bioelectrical impedance includes measuring the depth-specific bioelectrical impedance by adjusting a spacing of electrodes, an area of the electrodes, or a measurement frequency.

The measuring of the depth-specific bioelectrical impedance may include measuring a first bioelectrical impedance at a first depth by applying a first current to the object of interest via the first electrode and the second electrode and measuring a first voltage applied between the first electrode and the second electrode; and measuring a second bioelectrical impedance at a second depth by applying a second current to the object of interest via the third electrode and the fourth electrode and measuring a second voltage applied between the third electrode and the fourth electrode.

The measuring of the depth-specific bioelectrical impedance may include measuring a first bioelectrical impedance at a first depth by applying a first current to the object of interest via the first electrode and the second electrode and measuring a first voltage applied between the first electrode and the second electrode; connecting the first electrode and the third electrode; connecting the second electrode and the fourth electrode; and measuring a second bioelectrical impedance at a second depth by applying a second current to the object of interest via the first electrode that is connected to the third electrode and the second electrode that is connected to the fourth electrode and measuring a second voltage applied between the first electrode that is connected to the third electrode and the second electrode that is connected to the fourth electrode.

According to the invention, the method includes determining biometric information of the object of interest based on the optical signal and the depth-specific bioelectrical impedance.

The biometric information may include a body fat mass, a fat-free mass, a muscle mass, a skeletal muscle mass, a basal metabolic rate, an intracellular water mass, an extracellular water mass, a body water mass, an inorganic mass, a visceral fat mass, a blood flow volume, a calorie intake, a hematocrit, and a blood glucose level.

According to the invention, there is provided a method of measuring a biosignal that is performed by an apparatus for measuring the biosignal that comprises an optical sensor, and a first electrode and a second electrode that are spaced a predetermined distance apart from each other and are disposed symmetrically with respect to the optical sensor, the method may include measuring an optical signal of an object of interest by emitting light in a measurement region to the object of interest and receiving light reflected from the object of interest; and measuring a depth-specific bioelectrical impedance in the measurement region of the optical sensor using the first electrode and the second electrode.

The measuring of the depth-specific bioelectrical impedance may include measuring the depth-specific bioelectrical impedance by adjusting a measurement frequency.

The measuring of the depth-specific bioelectrical impedance may include measuring a first bioelectrical impedance at a first depth by applying a first current of a first frequency to the object of interest via the first electrode and the second electrode and measuring a first voltage applied between the first electrode and the second electrode; and measuring a second bioelectrical impedance at a second depth by applying a second current of a second frequency to the object of interest via the first electrode and the second electrode and measuring a second voltage applied between the first electrode and the second electrode.

The method may further include determining biometric information of the object of interest based on the optical signal and the depth-specific bioelectrical impedance.

Other features and aspects will be apparent from the following detailed description, the drawings, and the claims.

### BRIEF DESCRIPTION OF THE DRAWINGS

FIG. 1 is a block diagram illustrating an apparatus for measuring a biosignal according to an embodiment of the disclosure.
FIG. 2 is a block diagram illustrating a bioelectrical impedance sensor according to an embodiment of the disclosure.
FIG. 3 is a diagram illustrating an arrangement of an optical sensor and electrodes according to an embodiment of the disclosure.
FIG. 4 is a diagram illustrating a structure of the bioelectrical impedance sensor according to an embodiment of the disclosure.
FIG. 5 is a diagram for describing a penetration depth of energy in accordance with spacing between electrodes according to an embodiment of the disclosure.
FIG. 6 is a diagram illustrating a structure of the bioelectrical impedance sensor according to an embodiment of the disclosure.
FIG. 7 is a diagram illustrating a structure of the bioelectrical impedance sensor according to an embodiment of the disclosure.
FIG. 8 is a block diagram illustrating the bioelectrical impedance sensor according to an embodiment of the disclosure.
FIG. 9 is a diagram illustrating a structure of the bioelectrical impedance sensor according to an embodiment of the disclosure.
FIG. 10 is a block diagram illustrating an apparatus for measuring a biosignal according to an embodiment of the disclosure.
FIG. 11 is a block diagram illustrating an apparatus for measuring a biosignal according to an embodiment of the disclosure.
FIG. 12 is a flowchart illustrating a method of measuring a biosignal according to an embodiment of the disclosure.
FIG. 13 is a flowchart illustrating a method of measuring depth-specific bioelectrical impedance according to an embodiment of the disclosure.
FIG. 14 is a flowchart illustrating the method of measuring depth-specific bioelectrical impedance according to an embodiment of the disclosure.
FIG. 15 is a flowchart illustrating the method of measuring depth-specific bioelectrical impedance according to an embodiment of the disclosure.
FIG. 16 is a flowchart illustrating a method of measuring a biosignal according to an embodiment of the disclosure.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

Embodiments are described in greater detail below with reference to the accompanying drawings. In the following description, like drawing reference numerals are used for like elements, even in different drawings. The matters defined in the description, such as detailed construction and elements, are provided to assist in a comprehensive understanding of the embodiments. However, it is apparent that the embodiments can be practiced without those specifically defined matters. Also, well-known functions or constructions are not described in detail since they would obscure the description with unnecessary detail.

It should be noted that in some alternative implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. In some implementations, processes may include additional blocks, fewer blocks, different blocks, or differently arranged blocks than those depicted in the flowcharts. Additionally, or alternatively, two or more of the blocks may be performed in parallel.

Terms described below are selected by considering functions in the embodiment and meanings may vary depending on, for example, a user or operator's intentions or customs. Therefore, in the following embodiments, when terms are specifically defined, the meanings of the terms should be interpreted based on definitions, and otherwise, should be interpreted based on general meanings recognized by those skilled in the art.

It will be understood that, although the terms first, second, etc. may be used herein to describe various elements, these elements should not be limited by these terms. These terms are used to distinguish one element from another. As used herein, the singular forms are intended to include the plural forms as well, unless the context clearly indicates otherwise. It will be further understood that the terms "comprises" and/or "comprising," or "includes" and/or "including" when used in this description, specify the presence of stated features, numbers, steps, operations, elements, components or combinations thereof, but do not preclude the presence or addition of one or more other features, numbers, steps, operations, elements, components, or combinations thereof.

It will also be understood that the elements or components in the following description are discriminated in accordance with their respective main functions. In other words, two or more elements may be integrated into a single element or a single element may be divided into two or more elements in accordance with a subdivided function. Additionally, each of the elements in the following description may perform a sub-function or entirety of the function of another element as well as its main function, and some of the main functions of each of the elements may be performed exclusively by other elements. Each element may be realized in the form of a hardware component, a software component, a firmware component, and/or a combination thereof.

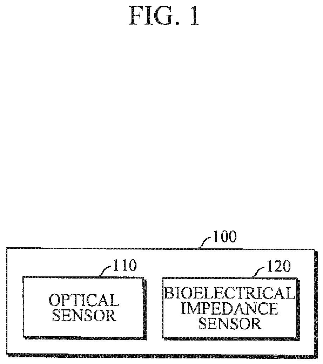

FIG. 1 is a block diagram illustrating an apparatus for measuring a biosignal according to an embodiment of the disclosure. The apparatus 100 for measuring a biosignal shown in FIG. 1 may be an apparatus capable of measuring an optical signal and impedance of an object of interest, and may be mounted in an electronic device. In this case, the electronic device may include a mobile phone, a smartphone, a tablet computer, a notebook computer, a personal digital assistant (PDA), a portable multimedia player (PMP), a navigation system, an MP3 player, a digital camera, a wearable device, and the like. The wearable device may include wearable devices of various types, such as a wristwatch type, a wrist band type, a ring type, a belt type, a necklace type, an ankle band type, a thigh-band type, a forearm band type, and the like. However, the electronic device and the wearable device are not limited to the above-described examples.

Referring to FIG. 1, the apparatus 100 for measuring a biosignal may include an optical sensor 110 and a bioelectrical impedance sensor 120.

The optical sensor 110 may measure an optical signal of the object of interest by emitting light to the object and receiving light reflected from the object of interest.

According to an embodiment, the optical sensor 110 may include a light source and a photodetector.

The light source may emit visible light rays or infrared rays to the object of interest. However, a wavelength of light emitted from the light source may vary according to the purpose of measurement. In addition, the light source is not necessarily configured with a single light emitting body and may be configured with a plurality of light-emitting bodies to form a light source array. In the case in which the light source is formed as a plurality of light-emitting bodies, the plurality of light-emitting bodies may emit light of different wavelengths or light of the same wavelength based on the purpose of measurement. According to an embodiment, the light source may include a light emitting diode (LED) or a laser diode, but is not limited thereto.

The photodetector may receive an optical signal reflected from the object of interest, and convert the optical signal into an electric signal. The photodetector is not necessarily formed as a single element, and may be formed as an array of a plurality of elements. According to an embodiment, the photodetector may include a photodiode, a photo transistor, a charge-coupled device (CCD), and the like, but is not limited thereto.

According to an embodiment, the optical sensor 110 may include a light source and a spectrometer. The light source is configured in a similar manner as described above and thus a detailed description thereof will not be reiterated.

The spectrometer may generate a spectrum of the object of interest by spectroscopically separating the light reflected from the object. According to an embodiment, the spectrometer may include an interferometric spectrometer, a grating spectrometer, a prism spectrometer, and the like, but is not limited thereto.

The bioelectrical impedance sensor 120 may measure a bioelectrical impedance of the object of interest. According to an embodiment, the bioelectrical impedance sensor 120 may measure depth-specific bioelectrical impedance in the same region as the measurement region of the optical sensor 110. To this end, the bioelectrical impedance sensor 120 may include a pair of electrodes disposed symmetrically with respect to the optical sensor 110.

According to an embodiment, the bioelectrical impedance sensor 120 may measure depth-specific bioelectrical impedance by adjusting a spacing between the electrodes, the area of the electrode, a measurement frequency, and the like.

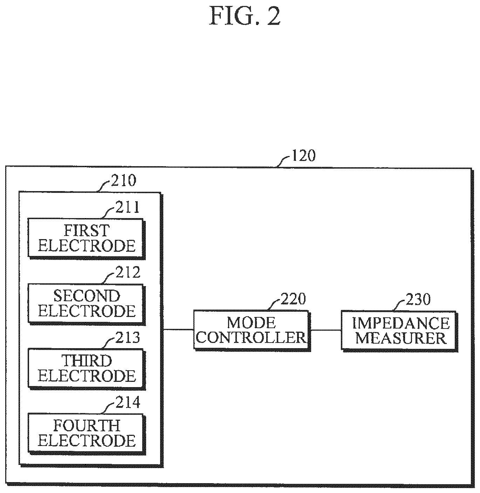

Hereinafter, the bioelectrical impedance sensor 120 will be described in more detail with reference to FIGS. 2 through 9.

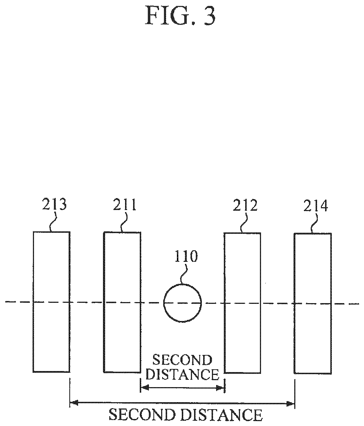

FIG. 2 is a block diagram illustrating the bioelectrical impedance sensor according to an embodiment of the disclosure, and FIG. 3 is a diagram illustrating an arrangement of an optical sensor and electrodes according to an embodiment of the disclosure.

Referring to FIG. 2, the bioelectrical impedance sensor 120 may include an electrode portion 210, a mode controller 220, and an impedance measurer 230.

The electrode portion 210 may include a first electrode 211, a second electrode 212, a third electrode 213, and a fourth electrode 214. In this case, the first electrode 211 and the second electrode 212 may be used to determine bioelectrical impedance information from regions relatively shallower than regions from which the third electrode 213 and the fourth electrode 214 are used to determine bioelectrical impedance information, and the third electrode 213 and the fourth electrode 214 may be used to determine bioelectrical impedance information of the regions relatively deeper than the regions from which the first electrode 211 and the second electrode 212 are used to determine bioelectrical impedance information.

Referring to FIG. 3, the first electrode 211 and the second electrode 212 may be spaced a first distance apart from each other, and may be arranged symmetrically with respect to the optical sensor 110. In addition, the third electrode 213 and the fourth electrode 214 may be spaced a second distance apart from each other, and may be arranged symmetrically with respect to the optical sensor 110. In this case, the second distance may be greater than the first distance, and the first electrode 211 and the second electrode 212 may be interposed between the third electrode 213 and the fourth electrode 214. That is, the optical sensor 110 may be centrally placed at a center, the third electrode 213 and the fourth electrode 214 may be placed outermost with respect to the center, the first electrode 211 and the second electrode 212 may each be disposed between the center and each of the outermost electrodes, and the respective centers of the optical sensor 110 and the four electrodes 211, 212, 213, and 214 may form a straight line.

Meanwhile, the four electrodes 211, 212, 213, and 214 may have the same size, but are not limited thereto. That is, the four electrodes 211, 212, 213, and 214 may be implemented to have different sizes according to the size of the apparatus 100 for measuring a biosignal and/or a desired depth at which bioelectrical impedance is to be measured.

The mode controller 220 may control an operation mode of the bioelectrical impedance sensor 120 to permit the bioelectrical impedance sensor 120 to measure bioelectrical impedance at each depth in the same region. For example, the mode controller 220 may set the operation mode of the bioelectrical impedance sensor 120 to a first mode or a second mode based on an input of a user's command, an occurrence of a predetermined event, and the like. In this case, the first mode may be a mode for measuring bioelectrical impedance at a first depth, and the second mode may be a mode for measuring bioelectrical impedance at a second depth that is different than the first depth.

The impedance measurer 230 may measure depth-specific bioelectrical impedance using the four electrodes 211, 212, 213, and 214 according to the operation mode set by the mode controller 220.

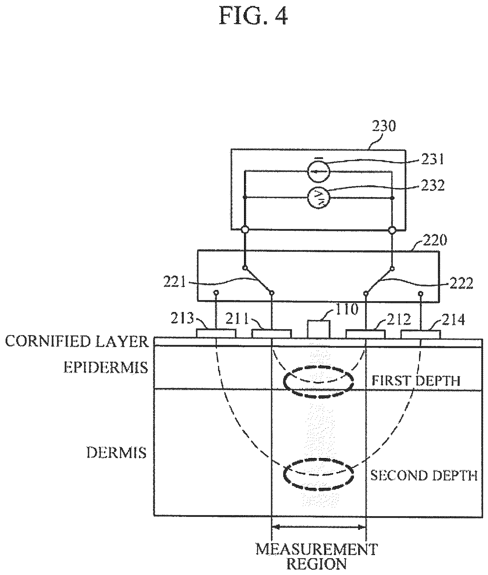

FIG. 4 is a diagram illustrating a structure of the bioelectrical impedance sensor according to an embodiment of the disclosure. FIG. 4 shows an embodiment in which a measurement depth can be varied by varying a distance between electrodes used for measurement, thereby utilizing the phenomenon that the penetration depth of electromagnetic wave signals increases as the spacing between the electrodes increases.

Referring to FIG. 4, the mode controller 220 may include a first switch 221 and a second switch 222. In the first mode, the first switch 221 may connect the impedance measurer 230 to the first electrode 211, and the second switch 222 may connect the impedance measurer 230 to the second electrode 212. In addition, in the second mode, the first switch 221 may connect the impedance measurer 230 to the third electrode 213, and the second switch 222 may connect the impedance measurer 230 to the fourth electrode 214.

The impedance measurer 230 may include a current source 231 and a voltmeter 232, may be connected to the first electrode 211 and the second electrode 212 via the first and second switches 221 and 222 in the first mode, and may be connected to the third electrode 213 and the fourth electrode 214 via the first and second switches 221 and 222 in the second mode.

In the first mode, the current source 231 may apply a current to the object of interest via the first and second electrodes 211 and 212, and the voltmeter 232 may measure a voltage (hereinafter referred to as a "first voltage") applied between the first electrode 211 and the second electrode 212 based on the current applied to the object by the current supply 231. At this time, the current applied via the first electrode 211 and the second electrode 212 may flow to a first depth, and, thus, the first voltage may include information up to the first depth.

In the second mode, the current source 231 may apply a current to the object of interest via the third electrode 213 and the fourth electrode 214, and the voltmeter 232 may measure a voltage (hereinafter referred to as a "second voltage") applied between the third electrode 213 and the fourth electrode 214 based on the current applied by the current source 231. At this time, the current applied via the third electrode 213 and the fourth electrode 214 may flow to a second depth, and, thus, the second voltage may include information up to the second depth.

Meanwhile, the impedance measurer 230 may calculate a bioelectrical impedance for the first depth of the object of interest based on the applied current and the measured first voltage associated with the first mode, and may calculate a bioelectrical impedance for the second depth of the object of interest based on the applied current and the measured second voltage associated with the second mode. As an example, the impedance measurer 230 may calculate a bioelectrical impedance using the relationship (i.e., V=I*Z) among voltage, current, and impedance.

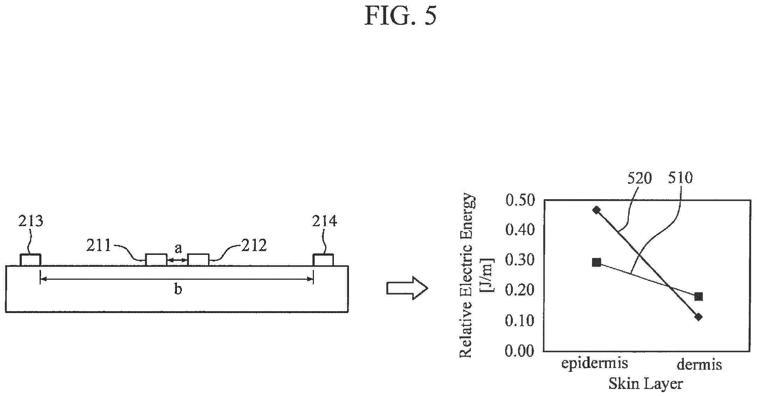

FIG. 5 is a diagram for describing a penetration depth of energy in accordance with a spacing between electrodes according to an embodiment of the disclosure.

When the first electrode 211 and the second electrode 212 are disposed at a first distance ("a") apart from each other, the third electrode 213 and the fourth electrode 214 are disposed at a second distance ("b") apart from each other (a < b), and a current is applied via the first electrode 211 and the second electrode 212, energy 520 transmitted to an outer layer is greater than energy 510 transmitted to a dermal layer. On the other hand, when a current is applied via the third electrode 213 and the fourth electrode 214, the energy 510 transmitted to the dermal layer is greater than the energy 520 transmitted to the outer layer. That is, the penetration depth of energy varies according to the spacing between the electrodes.

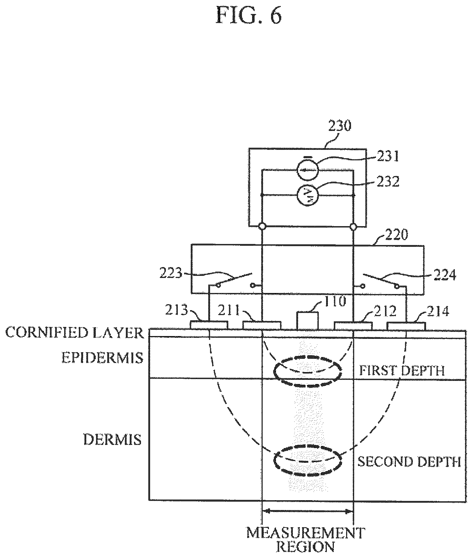

FIG. 6 is a diagram illustrating a structure of the bioelectrical impedance sensor according to an embodiment of the disclosure. FIG. 6 shows an embodiment in which a measurement depth can be varied by varying the area of the electrode used for measurement, thereby utilizing a phenomenon that the penetration depth of electromagnetic wave signals increases as the area of the electrode increases.

Referring to FIG. 6, the mode controller 220 may include a third switch 223 and a fourth switch 224. In the first mode, the third switch 223 may provide connection or disconnection of the connection between the first electrode 211 and the third electrode 213, and the fourth switch 224 may provide connection or disconnection of the connection between the second electrode 212 and the fourth electrode 214. In addition, in the second mode, the third switch 223 may connect the first electrode 211 and the third electrode 213, and the fourth switch 224 may connect the second electrode 212 and the fourth electrode 214. In the second mode, the first electrode 211 and the third electrode 213 are connected to form an integrated electrode and the second electrode 212 and the fourth electrode 214 are connected to form an integrated electrode, thereby increasing the area of the electrode.

The impedance measurer 230 may include the current source 231 and the voltmeter 232, and may be connected to the first electrode 211 and the second electrode 212.

In the first mode, the current source 231 may apply a current to an object of interest via the first electrode 211 and the second electrode 212, and the voltmeter 231 may measure a voltage (hereinafter referred to as a "third voltage") applied between the first electrode 211 and the second electrode 212 based on the current applied to the object by the current source 231. At this time, the current applied via the first electrode 211 and the second electrode 212 may flow to the first depth, and, thus, the third voltage may include information up to the first depth.

In the second mode, the current source 231 may apply a current to the object of interest via the first electrode 211 connected to the third electrode 213 and the second electrode 212 connected to the fourth electrode 214, and the voltmeter 231 may measure a voltage (hereinafter referred to as a "fourth voltage") applied between the first electrode 211 connected to the third electrode 213 and the second electrode 212 connected to the fourth electrode 214 based on the current applied by the current source 231. At this time, the current applied via the first electrode 211 connected to the third electrode 213 and the second electrode 212 connected to the fourth electrode 214 may flow to the second depth, and, thus, the fourth voltage may include information up to the second depth.

Meanwhile, the impedance measurer 230 may calculate a bioelectrical impedance for the first depth of the object of interest based on the applied current and the measured third voltage associated with the first mode, and calculate a bioelectrical impedance for the second depth of the object of interest based on the applied current and the measured fourth voltage associated with the second mode. At this time, the impedance measurer 230 may calculate a bioelectrical impedance using the relationship (i.e., V=I*Z) among voltage, current, and impedance.

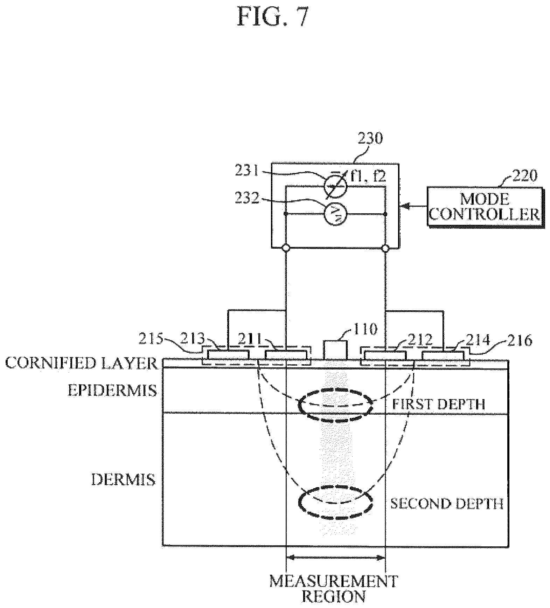

FIG. 7 is a diagram illustrating a structure of the bioelectrical impedance sensor according to an embodiment of the disclosure. FIG. 7 shows an embodiment in which the measurement depth can be varied by varying a measurement frequency, thereby utilizing a phenomenon that the penetration depth of electromagnetic waves changes with the change in the measurement frequency.

Referring to FIG. 7, the mode controller 220 may adjust a frequency of the current source 231 of the impedance measurer 230. For example, the mode controller 220 may set a frequency of the current source 231 to a first frequency ("f1") in a first mode, and set a frequency of the current source 231 to a second frequency ("f2") in a second mode. In this case, f1 may be different than f2.

The impedance measurer 230 may include the current source 231 and the voltmeter 232. In this case, the first electrode 211 and the third electrode 213 may be connected to form a fifth electrode 215, and the second electrode 212 and the fourth electrode 214 may be connected to form a sixth electrode 216.

In the first mode, the current source 231 may apply a current of the first frequency to the object of interest via the fifth electrode 215 and the sixth electrode 216, and the voltmeter 232 may measure a voltage (hereinafter referred to as a "fifth voltage") applied between the fifth electrode 215 and the sixth electrode 216 based on the current of the first frequency applied to the object by the current source 231. At this time, the current of the first frequency applied via the fifth electrode 215 and the sixth electrode 216 may flow to the first depth, and, thus, the fifth voltage may include information up to the first depth.

In the second mode, the current source 231 may apply a current of a second frequency to the object of interest via the fifth electrode 215 and the sixth electrode 216, and the voltmeter 232 may measure a voltage (hereinafter referred to as a "sixth voltage") applied between the fifth electrode 215 and the sixth electrode 216 based on the current of the second frequency applied by the current source 231. At this time, the current of the second frequency applied via the fifth electrode 215 and the sixth electrode 216 may flow to the second depth, and, therefore, the sixth voltage may include information up to the second depth.

Meanwhile, the impedance measurer 230 may calculate a bioelectrical impedance for the first depth of the object of interest based on the applied current and the measured fifth voltage associated with the first mode, and calculate a bioelectrical impedance for the second depth of the object based on the applied current and the measured sixth voltage associated with the second mode. At this time, the impedance measurer 230 may calculate a bioelectrical impedance using the relationship (i.e., V=I*Z) among voltage, current, and impedance.

Meanwhile, FIGS. 2 through 7 illustrate that the bioelectrical impedance sensor includes two pairs of electrodes, i.e., four electrodes, but the embodiments are not limited thereto. That is, the number of electrodes may vary according to the size of the apparatus for measuring a biosignal and/or the desired depth at which bioelectrical impedance is measured.

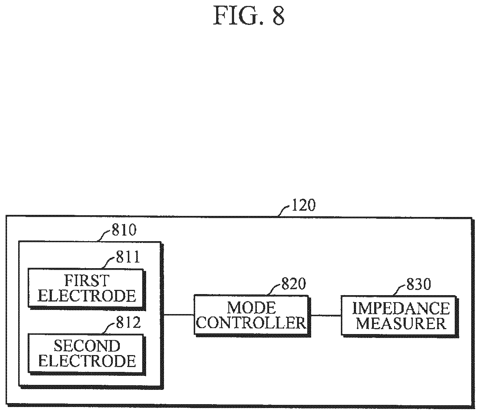

FIG. 8 is a block diagram illustrating the bioelectrical impedance sensor according to an embodiment of the disclosure.

Referring to FIG. 8, the bioelectrical impedance sensor 120 may include an electrode portion 810, a mode controller 820, and an impedance measurer 830.

The electrode portion 810 may include a first electrode 811 and a second electrode 812. In this case, the first electrode 211 and the second electrode 212 may be spaced a predetermined distance apart from each other, and may be disposed symmetrically with respect to the optical sensor 110. That is, the optical sensor 110 and the two electrodes 811 and 812 may form a straight line.

Meanwhile, the two electrodes 811 and 812 may have the same size, but are not limited thereto. That is, the two electrodes 811 and 812 may be implemented to have different sizes according to the size of the apparatus 100 for measuring a biosignal and/or a desired depth at which bioelectrical impedance is measured.

The mode controller 820 may control an operation mode of the bioelectrical impedance sensor 120 to permit the bioelectrical impedance sensor 120 to measure bioelectrical impedance at each depth in the same region. For example, the mode controller 820 may set the operation mode of the bioelectrical impedance sensor 120 to a first mode or a second mode based on an input of a user's command, an occurrence of a predetermined event, and the like. In this case, the first mode may be a mode for measuring bioelectrical impedance at a first depth, and the second mode may be a mode for measuring bioelectrical impedance at a second depth.

The impedance measurer 830 may measure depth-specific bioelectrical impedance using the two electrodes 811 and 812 according to the operation mode set by the mode controller 820.

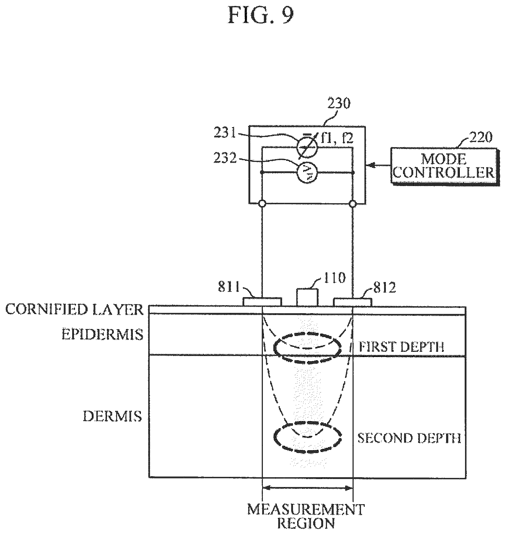

FIG. 9 is a diagram illustrating a structure of the bioelectrical impedance sensor according to an embodiment of the disclosure. FIG. 9 shows an embodiment in which a measurement depth can be varied by changing a measurement frequency with two electrodes, thereby utilizing a phenomenon that the penetration depth of electromagnetic waves changes with the change in the measurement frequency.

Referring to FIG. 9, the mode controller 820 may adjust a frequency of the current source 831. For example, the mode controller 820 may set a frequency of the current source 831 to a first frequency ("f1") in a first mode, and set the frequency of the current source 831 to a second frequency ("f2") in a second mode. In this case, f1 may be different than f2.

The impedance measurer 830 may include the current source 831 and a voltmeter 832.

In the first mode, the current source 831 may apply a current of the first frequency to the object of interest via a first electrode 811 and a second electrode 812, and the voltmeter 832 may measure a voltage (hereinafter referred to as a "seventh voltage") applied between the first electrode 811 and the second electrode 812 based on the current of the first frequency applied to the object of interest by the current source 831. At this time, the current of the first frequency applied via the first electrode 811 and the second electrode 812 may flow to the first depth, and, thus, the seventh voltage may include information up to the first depth.

In the second mode, the current source 831 may apply a current of a second frequency to the object of interest via the first electrode 811 and the second electrode 812, and the voltmeter 832 may measure a voltage (hereinafter referred to as a "eighth voltage") applied between the first electrode 811 and the second electrode 812 based on the current of the second frequency applied by the current source 831. At this time, the current of the second frequency applied via the first electrode 811 and the second electrode 812 may flow to the second depth, and, therefore, the eighth voltage may include information up to the second depth.

Meanwhile, the impedance measurer 830 may calculate a bioelectrical impedance for the first depth of the object of interest based on the applied current and the measured seventh voltage associated with the first mode, and calculate a bioelectrical impedance for the second depth of the object based on the applied current applied and the measured eighth voltage associated with the second mode. As an example, the impedance measurer 230 may calculate a bioelectrical impedance using the relationship (i.e., V=I*Z) among voltage, current, and impedance.

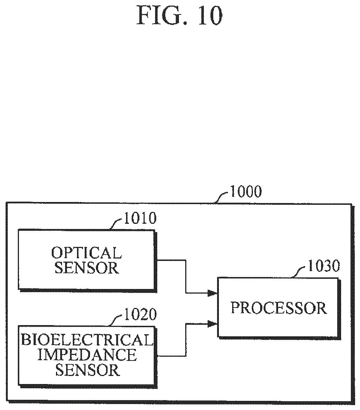

FIG. 10 is a block diagram illustrating an apparatus for measuring a biosignal according to an embodiment of the disclosure. The apparatus 1000 for measuring a biosignal shown in FIG. 10 may be mounted in an electronic device. In this case, the electronic device may include a mobile phone, a smartphone, a tablet computer, a notebook computer, a PDA, a PMP, a navigation system, an MP3 player, a digital camera, a wearable device, and the like. The wearable device may include wearable devices of various types, such as a wristwatch type, a wrist band type, a ring type, a belt type, a necklace type, an ankle band type, a thigh-band type, a forearm band type, and the like. However, the electronic device and the wearable device are not limited to the above-described examples.

Referring to FIG. 10, the apparatus 1000 for measuring a biosignal may include an optical sensor 1010, a bioelectrical impedance sensor 1020, and a processor 1030. The optical sensor 1010 and the bioelectrical impedance sensor 1020 may be configured in a similar manner as the optical sensor 110 and the bioelectrical impedance sensor 120 shown in FIG. 1, respectively, and thus detailed descriptions thereof will not be reiterated.

The processor 1030 may determine biometric information of an object of interest based on an optical signal measured by the optical sensor 1010 and depth-specific bioelectrical impedance measured by the bioelectrical impedance sensor 1020. In some implementations, the biometric information may include body fat mass, fat-free mass, muscle mass, skeletal muscle mass, basal metabolic rate, intracellular water mass, extracellular water mass, body water mass, inorganic mass, visceral fat mass, blood flow volume, calorie intake, hematocrit, blood glucose, and the like. According to the invention, the processor 1030 determines the biometric information of the object of interest using a biometric information estimation model. The biometric information estimation model defines a relationship between an optical signal, a depth-specific bioelectrical impedance, and biometric information, and may be established in advance through regression analysis or machine learning, and stored in an internal or external database of the processor 1030. The biometric information estimation model may be established in the form of a mathematical algorithm or a matching table, but is not limited thereto. In some implementations, the machine learning techniques may include, for example, supervised and/or unsupervised techniques, such as artificial networks, Bayesian statistics, learning automata, Hidden Markov Modeling, linear classifiers, quadratic classifiers, decision trees, association rule learning, or the like. The biometric information estimation model may receive an input including information associated with the optical signal and the depth-specific bioelectrical impedance, and generate an output that identifies the biometric information.

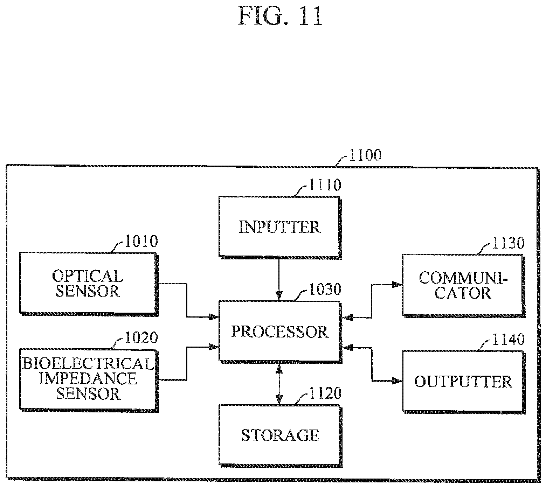

FIG. 11 is a block diagram illustrating an apparatus for measuring a biosignal according to an embodiment of the disclosure. The apparatus 1100 for measuring a biosignal shown in FIG. 11 may be mounted in an electronic device. In this case, the electronic device may include a mobile phone, a smartphone, a tablet computer, a notebook computer, a PDA, a PMP, a navigation system, an MP3 player, a digital camera, a wearable device, and the like. The wearable device may include wearable devices of various types, such as a wristwatch type, a wrist band type, a ring type, a belt type, a necklace type, an ankle band type, a thigh-band type, a forearm band type, and the like. However, the electronic device and the wearable device are not limited to the aforementioned examples.

Referring to FIG. 11, the apparatus 1100 for measuring a biosignal may include an optical sensor 1010, a bioelectrical impedance sensor 1020, a processor 1030, an input component 1110, a storage 1120, a communication interface 1130, and an output component 1140. The optical sensor 1010, the bioelectrical impedance sensor 1020, and the processor 1030 may be configured in a similar manner as described above with reference to FIG. 10, and thus detailed descriptions thereof will not be reiterated.

The input component 1110 may receive various operation signals. For example, input component 1110 includes a component that permits the apparatus 1100 to receive information, such as via user input (e.g., a touch screen display, a keyboard, a keypad, a mouse, a button, a switch, and/or a microphone). Additionally, or alternatively, the input component 1110 may include a sensor for sensing information (e.g., a global positioning system (GPS) component, an accelerometer, a gyroscope, and/or an actuator). According to an embodiment, the input component 1110 may include a key pad, a dome switch, a touch pad (e.g., a resistive/capacitive touch pad), a jog wheel, a jog switch, a hardware button, and the like. In particular, when a touch pad has a layered structure with a display, the structure may be referred to as a touch screen.

Programs or commands for operation of the apparatus 1100 for measuring a biosignal may be stored in the storage 1120, and data input to and output from the apparatus 1100 may also be stored in the storage 1120. In addition, data processed by the apparatus 1100 and data (e.g., a biometric information estimation model, and the like) utilized by the apparatus 1100 to perform data processing may be stored in the storage 1120.

The storage 1120 may include a type of storage medium, such as a flash memory type, a hard disk type, a multimedia card micro type, a card type memory (e.g., SD or XD memory) random access memory (RAM), static random access memory (SRAM), read-only memory (ROM), electrically erasable programmable read-only memory (EEPROM), programmable read-only memory (PROM), a magnetic memory, a magnetic disk, an optical disk, and the like. In addition, the apparatus 1100 for measuring a biosignal may access an external storage medium, such as a web storage that performs the storage function of the storage 1120 on the Internet.

The communication interface 1130 may communicate with an external device. For example, the communication interface 1130 may transmit data utilized by the apparatus 1100 or processing result data of the apparatus 1100 to the external device, and/or receive data for that permits the measurement of a biosignal and/or the estimation of biometric information from the external device.

The external device may be medical equipment which uses the data utilized by the apparatus 1100 or the processing result data of the apparatus 1100, a printer, a display device configured to output a result, and the like. In addition, the external device may be a digital TV, a desktop computer, a mobile phone, a smartphone, a tablet computer, a PDA, a PMP, a navigation system, an MP3 player, a digital camera, a wearable device, or the like, but is not limited thereto.
The communication interface 1130 may communicate with the external device using, for example, Bluetooth, Bluetooth low energy (BLE) communication, near-field communication (NFC), wireless local access network (WLAN) communication, ZigBee communication, infrared data association (IrDA) communication, Wi-Fi direct (WFD) communication, ultra-wideband (UWB) communication, Ant+ communication, Wi-Fi communication, radio frequency identification (RFID) communication, third generation (3G) communication, fourth generation (4G) communication, fifth generation (5G) communication, and the like. For example, communication interface 1130 may include an Ethernet interface, an optical interface, a coaxial interface, an infrared interface, a radio frequency (RF) interface, a universal serial bus (USB) interface, a Wi-Fi interface, a cellular network interface, or the like.

However, these are merely examples and the embodiment is not limited thereto.

The output component 1140 may output the data utilized by the apparatus 1100 or the processing result data of the apparatus 1100. According to an embodiment, the output component 1140 may output the data utilized by the apparatus 1100 or the processing result data of the apparatus 1100 using an audible method, a visual method, a tactile method, and the like. For example, the output component 1140 may include a display, a speaker, a vibrator, and the like. As used herein, the term "component" is intended to be broadly construed as hardware, firmware, or a combination of hardware and software.

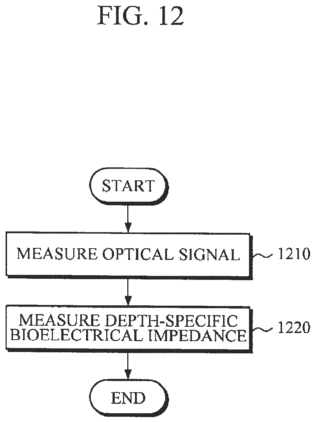

FIG. 12 is a flowchart illustrating a method of measuring a biosignal according to an embodiment of the disclosure. The method shown in FIG. 12 may be performed by the apparatus for measuring a biosignal shown in FIG. 1.

Referring to FIG. 12, an apparatus for measuring a biosignal may measure an optical signal using an optical sensor by emitting light to an object of interest and receiving light reflected from the object of interest, in operation 1210.

In operation 1220, the apparatus for measuring a biosignal may measure depth-specific bioelectrical impedance in the same region as a measurement region of the optical sensor. For example, the apparatus for measuring a biosignal may use a set of electrodes which are disposed symmetrically with respect to the optical sensor to adjust a spacing of the electrodes, an area of the electrodes, and/or a measurement frequency, thereby measuring the depth-specific bioelectrical impedance in the same region as the measurement region of the optical sensor.

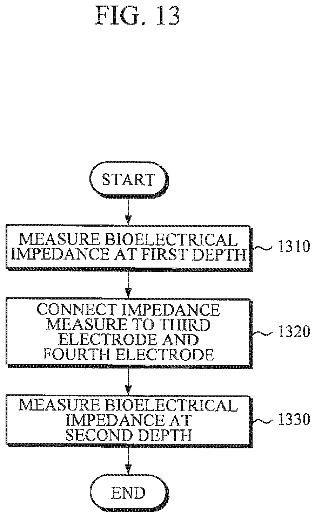

FIG. 13 is a flowchart illustrating a method of measuring depth-specific bioelectrical impedance according to an embodiment of the disclosure. The method shown in FIG. 13 may be an embodiment of the operation 1220 for measuring the depth-specific bioelectrical impedance of FIG. 12.

Referring to FIG. 13, an apparatus for measuring a biosignal may measure bioelectrical impedance at a first depth using first and second electrodes which are disposed at a first distance apart from each other and are arranged symmetrically with respect to an optical sensor, in operation 1310. For example, the apparatus for measuring a biosignal may apply a current to an object of interest via the first and second electrodes by connecting an impedance measurer to the first and second electrodes, and measure a voltage applied between the first and second electrodes based on the current applied to the object of interest, thereby measuring the bioelectrical impedance at the first depth.

In operation 1320, the apparatus for measuring a biosignal may connect the impedance measurer to a third electrode and a fourth electrode, which are disposed at a second distance apart from each other and are arranged symmetrically with respect to the optical sensor.

In operation 1330, the apparatus for measuring a biosignal may measure a bioelectrical impedance at a second depth using the third and fourth electrodes. For example, the apparatus for measuring a biosignal may apply a current to the object of interest via the third and fourth electrodes, and measure a voltage applied between the third and fourth electrodes based on the current applied to the object of interest, thereby measuring the bioelectrical impedance at the second depth.

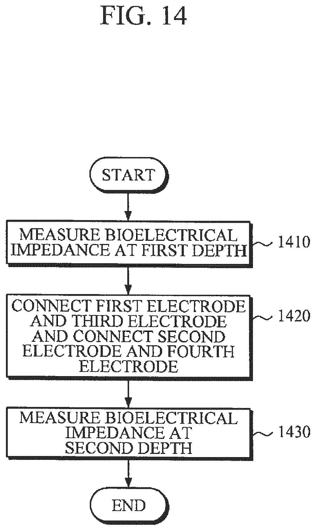

FIG. 14 is a flowchart illustrating the method of measuring depth-specific bioelectrical impedance according to an embodiment of the disclosure. The method shown in FIG. 14 may be an embodiment of the operation 1220 for measuring the depth-specific bioelectrical impedance of FIG. 12.

Referring to FIG. 14, an apparatus for measuring a biosignal may measure bioelectrical impedance at a first depth using a first electrode and a second electrode, which are disposed at a first distance apart from each other and are arranged symmetrically with respect to an optical sensor, in operation 1410. For example, the apparatus for measuring a biosignal may apply a current to an object of interest via the first and second electrodes, and measure a voltage applied between the first and second electrodes based on the current applied to the object of interest, thereby measuring the bioelectrical impedance at the first depth.

In operation 1420, the apparatus for measuring a biosignal may connect the first electrode to a third electrode, and connect the second electrode to a fourth electrode, wherein the third electrode and the fourth electrode are disposed at a second distance apart from each other and are arranged symmetrically with respect to the optical sensor.

The apparatus for measuring a biosignal may measure bioelectrical impedance at a second depth using the first electrode connected to the third electrode and the second electrode connected to the fourth electrode, in operation 1430. For example, the apparatus for measuring a biosignal may apply a current to the object of interest via the first electrode connected to the third electrode and the second electrode connected to the fourth electrode, and measure a voltage applied between the first electrode connected to the third electrode and the second electrode connected to the fourth electrode based on the current applied to the object, thereby measuring the bioelectrical impedance at the second depth.

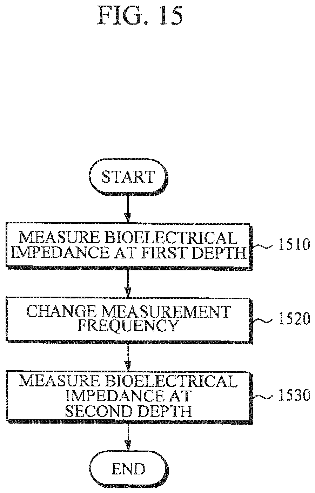

FIG. 15 is a flowchart illustrating the method of measuring depth-specific bioelectrical impedance according to an embodiment of the disclosure. The method shown in FIG. 15 may be an embodiment of the operation 1220 for measuring the depth-specific bioelectrical impedance of FIG. 12.

Referring to FIG. 15, an apparatus for measuring a biosignal may measure a bioelectrical impedance at a first depth using a first frequency and using a fifth electrode and a sixth electrode, which are disposed at a first distance apart from each other and are arranged symmetrically with respect to an optical sensor, in operation 1510. For example, the apparatus for measuring a biosignal may apply a current of the first frequency to an object of interest via the fifth and sixth electrodes, and measure a voltage applied between the fifth and sixth electrodes based on the current applied to the object of interest, thereby measuring the bioelectrical impedance at the first depth.

In operation 1520, the apparatus for measuring a biosignal may change a measurement frequency of an impedance measurer from the first frequency to a second frequency that is different than the first frequency.

In operation 1530, the apparatus for measuring a biosignal may measure a bioelectrical impedance at a second depth using a second frequency and using the fifth and sixth electrodes. For example, the apparatus for measuring a biosignal may apply a current of the second frequency to the object of interest via the fifth and sixth electrodes, and measure a voltage applied between the fifth and sixth electrodes based on the current applied to the object, thereby measuring the bioelectrical impedance at the second depth.

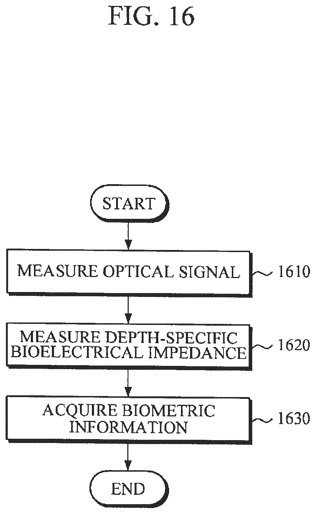

FIG. 16 is a flowchart illustrating a method of measuring a biosignal according to an embodiment of the disclosure. The method shown in FIG. 16 may be performed by the apparatus for measuring a biosignal shown in FIG. 10.

Referring to FIG. 16, an apparatus for measuring a biosignal may measure an optical signal of an object of interest using an optical sensor by emitting light to the object and receiving light reflected from the object of interest, in operation 1610.

In operation 1620, the apparatus for measuring a biosignal may measure a depth-specific bioelectrical impedance in the same region as a measurement region of the optical sensor. For example, the apparatus for measuring a biosignal may use a set of electrodes which are disposed symmetrically with respect to the optical sensor to adjust at a spacing of the electrodes, the area of the electrodes, and/or a measurement frequency, thereby measuring the depth-specific bioelectrical impedance in the same region as the measurement region of the optical sensor.

In operation 1630, the apparatus for measuring a biosignal may determine biometric information of the object of interest based on the measured optical signal and the measured depth-specific bioelectrical impedance. As examples, the biometric information may include body fat mass, fat-free mass, muscle mass, skeletal muscle mass, basal metabolic rate, intracellular water mass, extracellular water mass, body water mass, inorganic mass, visceral fat mass, blood flow volume, calorie intake, hematocrit, blood glucose, and the like. According to the invention, the apparatus for measuring a biosignal determines the biometric information of the object using a biometric information estimation model. The biometric information estimation model defines a relationship between an optical signal, a depth-specific bioelectrical impedance, and biometric information, and may be established in advance through regression analysis or machine learning, and may be stored in an internal or external database of the apparatus for measuring a biosignal. The biometric information estimation model may be established in the form of a mathematical algorithm or a matching table, but is not limited thereto.

The embodiments can be implemented as computer readable instructions in a computer readable recording medium. Code and code segments constituting computer programs can be easily inferred by a skilled computer programmer in the art. The computer readable recording medium may include a non-transitory computer readable medium. The computer readable recording medium includes all types of recording media in which computer readable data is stored. Examples of the computer readable recording medium include a ROM, a RAM, a CD-ROM, a magnetic tape, a floppy disk, and an optical data storage. Further, the recording medium may be implemented in the form of a carrier wave such as Internet transmission. In addition, the computer readable recording medium may be distributed to computer systems via a network, in which computer readable instructions may be stored and executed in a distributed manner.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations may be within the scope of the following claims.

## Claims

1. An apparatus (100, 1000, 1100) for measuring a biosignal, comprising:
an optical sensor (110, 1010) configured to emit light in a measurement region to an object of interest, and receive an optical signal reflected from the object of interest;
a bioelectrical impedance sensor (120, 1020) configured to measure a depth-specific bioelectrical impedance in the measurement region of the optical sensor, wherein the bioelectrical impedance sensor comprises:
at least a first electrode and a second electrode which are spaced a predetermined distance apart from each other and are disposed symmetrically with respect to the optical sensor; and
an impedance measurer configured to measure a first bioelectrical impedance at a first depth in the measurement region of the optical sensor and measure a second bioelectrical impedance at a second depth in the measurement region of the optical sensor, wherein the first depth and the second depth are different from each other, and wherein the bioelectrical impedance sensor is configured to measure the first bioelectrical impedance at the first depth and the second bioelectrical impedance at the second depth by adjusting a spacing of the electrodes, an area of the electrodes, or a measurement frequency; and
the apparatus further comprises:
a processor (1030) configured to determine biometric information of the object of interest based on the received optical signal and the depth-specific bioelectrical impedance by using a biometric information estimation model, wherein the biometric information estimation model defines a relationship between the optical signal, the depth-specific bioelectrical impedance and the biometric information.

2. The apparatus of claim 1, wherein the bioelectrical impedance sensor comprises:
a third electrode and a fourth electrode that are spaced a second distance apart from each other and are disposed symmetrically with respect to the optical sensor; and
wherein the impedance measurer is further configured to:
measure the first bioelectrical impedance at the first depth using the first electrode and the second electrode; and
measure the second bioelectrical impedance at the second depth using the third electrode and the fourth electrode.

3. The apparatus of claim 2, wherein the first electrode and the second electrode are interposed between the third electrode and the fourth electrode; and/or
wherein respective centers of the first electrode, the second electrode, the third electrode, the fourth electrode, and the optical sensor form a straight line.

4. The apparatus of claim 2, wherein the bioelectrical impedance sensor further comprises:
a mode controller configured to control an operation mode for measuring the first bioelectrical impedance at the first depth and the second bioelectrical impedance at the second depth.

5. The apparatus of claim 4, wherein the mode controller is configured to:
connect the impedance measurer to the first electrode and the second electrode in a first operation mode; and
connect the impedance measurer to the third electrode and the fourth electrode in a second operation mode, preferably,
wherein the impedance measurer comprises:
a current source configured to:
apply a first current to the object of interest via the first electrode and the second electrode in the first operation mode; and
apply a second current to the object of interest via the third electrode and the fourth electrode in the second operation mode; and
a voltmeter configured to:
measure a first voltage applied between the first electrode and the second electrode in the first operation mode; and
measure a second voltage applied between the third electrode and the fourth electrode in the second operation mode.

6. The apparatus of claim 4 or 5, wherein the mode controller is configured to:
disconnect the first electrode and the third electrode in a first operation mode,
disconnect the second electrode and the fourth electrode in the first operation mode,
connect the first electrode and the third electrode in a second operation mode, and
connect the second electrode and the fourth electrode in the second operation mode.

7. The apparatus of claim 6, wherein the impedance measurer comprises:
a current source configured to:
apply a first current to the object of interest via the first electrode and the second electrode in the first operation mode, and
apply a second current to the object of interest via the third electrode that is connected to the first electrode and via the fourth electrode that is connected to the second electrode in the second operation mode; and
a voltmeter configured to:
measure a first voltage applied between the first electrode and the second electrode in the first operation mode, and
measure a second voltage applied between the third electrode that is connected to the first electrode and the fourth electrode that is connected to the second electrode in the second operation mode.

8. The apparatus of claim 1, wherein the bioelectrical impedance sensor further comprises:
a mode controller configured to control an operation mode for measuring the depth-specific bioelectrical impedance, preferably,
wherein the mode controller is further configured to:
set the measurement frequency to a first frequency in a first operation mode, and
set the measurement frequency to a second frequency in a second operation mode.

9. The apparatus of claim 8, wherein the impedance measurer comprises:
a current source configured to:
apply a first current of the first frequency to the object of interest via the first electrode and the second electrode in the first operation mode, and
apply a second current of the second frequency to the object of interest via the first electrode and the second electrode in the second operation mode; and
a voltmeter configured to measure a voltage applied between the first electrode and the second electrode.

10. The apparatus of claim one of claims 1 to 9, wherein the biometric information includes a body fat mass, a fat-free mass, a muscle mass, a skeletal muscle mass, a basal metabolic rate, an intracellular water mass, an extracellular water mass, a body water mass, an inorganic mass, a visceral fat mass, a blood flow volume, a calorie intake, a hematocrit, or a blood glucose level.

11. A method of measuring a biosignal which is performed by an apparatus for measuring the biosignal that comprises an optical sensor, a first electrode and a second electrode that are spaced a first distance apart from each other and are disposed symmetrically with respect to the optical sensor, and a third electrode and a fourth electrode that are spaced a second distance apart from each other and are disposed symmetrically with respect to the optical sensor, the method comprising:
measuring an optical signal by emitting light in a measurement region to an object of interest and receiving light reflected from the object of interest;
measuring a depth-specific bioelectrical impedance in the measurement region of the optical sensor using the first electrode, the second electrode, the third electrode, and the fourth electrode, wherein the measuring of the depth-specific bioelectrical impedance comprises measuring the depth-specific bioelectrical impedance by adjusting a spacing of the electrodes, an area of the electrodes, or a measurement frequency, and
wherein the measuring of the depth-specific bioelectrical impedance further comprises measuring a first bioelectrical impedance at a first depth in the measurement region of the optical sensor and measuring a second bioelectrical impedance at a second depth in the measurement region of the optical sensor, and wherein the first depth and the second depth are different from each other; and
determining biometric information of the object of interest based on the optical signal and the depth-specific bioelectrical impedance by using a biometric information estimation model, wherein the biometric information estimation model defines a relationship between the optical signal, the depth-specific bioelectrical impedance and the biometric information.

12. The method of claim 11, wherein the measuring of the depth-specific bioelectrical impedance comprises:
measuring a first bioelectrical impedance at a first depth by applying a first current to the object of interest via the first electrode and the second electrode and measuring a first voltage applied between the first electrode and the second electrode; and
measuring a second bioelectrical impedance at a second depth by applying a second current to the object of interest via the third electrode and the fourth electrode and measuring a second voltage applied between the third electrode and the fourth electrode.

13. The method of claim 11, wherein the measuring of the depth-specific bioelectrical impedance comprises:
measuring a first bioelectrical impedance at a first depth by applying a first current to the object of interest via the first electrode and the second electrode and measuring a first voltage applied between the first electrode and the second electrode;
connecting the first electrode and the third electrode;
connecting the second electrode and the fourth electrode; and
measuring a second bioelectrical impedance at a second depth by applying a second current to the object of interest via the first electrode that is connected to the third electrode and the second electrode that is connected to the fourth electrode and measuring a second voltage applied between the first electrode that is connected to the third electrode and the second electrode that is connected to the fourth electrode.

14. The method of claim 11, wherein the biometric information includes a body fat mass, a fat-free mass, a muscle mass, a skeletal muscle mass, a basal metabolic rate, an intracellular water mass, an extracellular water mass, a body water mass, an inorganic mass, a visceral fat mass, a blood flow volume, a calorie intake, a hematocrit, or a blood glucose level.

## Patentansprüche

1. Eine Vorrichtung (100, 1000, 1100) zum Messen eines Biosignals, umfassend:
einen optischen Sensor (110, 1010), der dazu konfiguriert ist, Licht in einem Messbereich zu einem Objekt von Interesse auszusenden und ein optisches Signal zu empfangen, das von dem Objekt von Interesse reflektiert wird;
einen bioelektrischen Impedanzsensor (120, 1020), der dazu konfiguriert ist, eine tiefenspezifische bioelektrische Impedanz im Messbereich des optischen Sensors zu messen, wobei der bioelektrische Impedanzsensor umfasst:
mindestens eine erste Elektrode und eine zweite Elektrode, die in einem vorbestimmten Abstand von einander beabstandet und symmetrisch in Bezug auf den optischen Sensor angeordnet sind; und
ein Impedanzmesser, der dazu konfiguriert ist, eine erste bioelektrische Impedanz in einer ersten Tiefe im Messbereich des optischen Sensors zu messen und eine zweite bioelektrische Impedanz in einer zweiten Tiefe im Messbereich des optischen Sensors zu messen, wobei die erste Tiefe und die zweite Tiefe voneinander verschieden sind, und wobei der bioelektrische Impedanzsensor dazu konfiguriert ist, die erste bioelektrische Impedanz in der ersten Tiefe und die zweite bioelektrische Impedanz in der zweiten Tiefe zu messen, indem er einen Abstand der Elektroden, eine Fläche der Elektroden oder eine Messfrequenz einstellt; und
die Vorrichtung umfasst ferner:
einen Prozessor (1030), der dazu konfiguriert ist, biometrische Informationen des Objekts von Interesse auf der Grundlage des empfangenen optischen Signals und der tiefenspezifischen bioelektrischen Impedanz unter Verwendung eines Modells zur Schätzung biometrischer Informationen zu bestimmen, wobei das Modell zur Schätzung biometrischer Informationen eine Beziehung zwischen dem optischen Signal, der tiefenspezifischen bioelektrischen Impedanz und den biometrischen Informationen definiert.

2. Die Vorrichtung nach Patentanspruch 1, wobei der bioelektrische Impedanzsensor umfasst:
eine dritte Elektrode und eine vierte Elektrode, die in einem zweiten Abstand von einander beabstandet und symmetrisch in Bezug auf den optischen Sensor angeordnet sind; und
wobei der Impedanzmesser ferner dazu konfiguriert ist:
die erste bioelektrische Impedanz in der ersten Tiefe unter Verwendung der ersten Elektrode und der zweiten Elektrode zu messen; und
die zweite bioelektrische Impedanz in der zweiten Tiefe unter Verwendung der dritten Elektrode und der vierten Elektrode zu messen.

3. Die Vorrichtung nach Patentanspruch 2, wobei die erste Elektrode und die zweite Elektrode zwischen der dritten Elektrode und der vierten Elektrode angeordnet sind; und/oder
wobei die jeweiligen Mittelpunkte der ersten Elektrode, der zweiten Elektrode, der dritten Elektrode, der vierten Elektrode und des optischen Sensors eine gerade Linie bilden.

4. Die Vorrichtung nach Patentanspruch 2, wobei der bioelektrische Impedanzsensor weiterhin umfasst:
eine Betriebsartsteuerung, die dazu konfiguriert ist, eine Betriebsart zum Messen der ersten bioelektrischen Impedanz in der ersten Tiefe und der zweiten bioelektrischen Impedanz in der zweiten Tiefe zu steuern.

5. Die Vorrichtung nach Patentanspruch 4, wobei die Betriebsartsteuerung dazu konfiguriert ist:
den Impedanzmesser in einer ersten Betriebsart an die erste Elektrode und die zweite Elektrode anzuschließen; und
den Impedanzmesser in einer zweiten Betriebsart an die dritte und vierte Elektrode anzuschließen,
wobei der Impedanzmesser vorzugsweise umfasst:
eine Stromquelle, die dazu konfiguriert ist:
einen ersten Strom an das Objekt von Interesse über die erste Elektrode und die zweite Elektrode in der ersten Betriebsart anzulegen; und
einen zweiten Strom an das Objekt von Interesse über die dritte Elektrode und die vierte Elektrode in der zweiten Betriebsart anzulegen; und
ein Voltmeter, das dazu konfiguriert ist:
eine erste Spannung zu messen, die zwischen der ersten Elektrode und der zweiten Elektrode in der ersten Betriebsart anliegt; und
eine zweite Spannung zu messen, die zwischen der dritten Elektrode und der vierten Elektrode in der zweiten Betriebsart anliegt.

6. Die Vorrichtung nach Patentanspruch 4 oder 5, wobei die Betriebsartsteuerung dazu konfiguriert ist:
die erste Elektrode und die dritte Elektrode in einer ersten Betriebsart zu trennen,
die zweite Elektrode und die vierte Elektrode in der ersten Betriebsart zu trennen,
die erste Elektrode und die dritte Elektrode in einer zweiten Betriebsart zu verbinden, und
die zweite Elektrode und die vierte Elektrode in der zweiten Betriebsart zu verbinden.

7. Die Vorrichtung nach Patentanspruch 6, wobei der Impedanzmesser umfasst:
eine Stromquelle, die dazu konfiguriert ist:
einen ersten Strom an das Objekt von Interesse über die erste Elektrode und die zweite Elektrode in der ersten Betriebsart anzulegen, und
einen zweiten Strom an das Objekt von Interesse über die dritte Elektrode, die mit der ersten Elektrode verbunden ist, und über die vierte Elektrode, die mit der zweiten Elektrode verbunden ist, in der zweiten Betriebsart anzulegen; und
ein Voltmeter, das dazu konfiguriert ist:
eine erste Spannung zu messen, die zwischen der ersten Elektrode und der zweiten Elektrode in der ersten Betriebsart anliegt, und
eine zweite Spannung zu messen, die zwischen der dritten Elektrode, die mit der ersten Elektrode verbunden ist, und der vierten Elektrode, die mit der zweiten Elektrode verbunden ist, in der zweiten Betriebsart anliegt.

8. Die Vorrichtung nach Patentanspruch 1, wobei der bioelektrische Impedanzsensor weiterhin umfasst:
eine Betriebsartsteuerung, die dazu konfiguriert ist, eine Betriebsart zum Messen der tiefenspezifischen bioelektrischen Impedanz zu steuern,
wobei die Betriebsartsteuerung vorzugsweise ferner dazu konfiguriert ist:
die Messfrequenz auf eine erste Frequenz in einer ersten Betriebsart einzustellen, und
die Messfrequenz auf eine zweite Frequenz in einer zweiten Betriebsart einzustellen.

9. Die Vorrichtung nach Patentanspruch 8, wobei der Impedanzmesser umfasst:
eine Stromquelle, die dazu konfiguriert ist:
einen ersten Strom mit der ersten Frequenz an das Objekt von Interesse über die erste Elektrode und die zweite Elektrode in der ersten Betriebsart anzulegen, und
einen zweiten Strom mit der zweiten Frequenz an das Objekt von Interesse über die erste Elektrode und die zweite Elektrode in der zweiten Betriebsart anzulegen; und
ein Voltmeter, das dazu konfiguriert ist, eine zwischen der ersten Elektrode und der zweiten Elektrode angelegte Spannung zu messen.

10. Die Vorrichtung nach einem der Patentansprüche 1 bis 9, wobei die biometrischen Informationen eine Körperfettmasse, eine fettfreie Masse, eine Muskelmasse, eine Skelettmuskelmasse, eine Grundumsatzrate, eine intrazelluläre Wassermasse, eine extrazelluläre Wassermasse, eine Körperwassermasse, eine anorganische Masse, eine viszerale Fettmasse, ein Blutflussvolumen, eine Kalorienaufnahme, ein Hämatokrit oder einen Blutzuckerspiegel umfassen.

11. Ein Verfahren zum Messen eines Biosignals, das von einer Vorrichtung zum Messen des Biosignals durchgeführt wird, die einen optischen Sensor, eine erste Elektrode und eine zweite Elektrode, die in einem ersten Abstand voneinander beabstandet und symmetrisch in Bezug auf den optischen Sensor angeordnet sind, und eine dritte Elektrode und eine vierte Elektrode umfasst, die in einem zweiten Abstand voneinander beabstandet und symmetrisch in Bezug auf den optischen Sensor angeordnet sind, wobei das Verfahren umfasst:
Messen eines optischen Signals durch Aussenden von Licht in einem Messbereich zu einem Objekt von Interesse und Empfangen von Licht, das von dem Objekt von Interesse reflektiert wird;
Messen einer tiefenspezifischen bioelektrischen Impedanz in dem Messbereich des optischen Sensors unter Verwendung der ersten Elektrode, der zweiten Elektrode, der dritten Elektrode und der vierten Elektrode, wobei das Messen der tiefenspezifischen bioelektrischen Impedanz das Messen der tiefenspezifischen bioelektrischen Impedanz durch Einstellen eines Abstands der Elektroden, einer Fläche der Elektroden oder einer Messfrequenz umfasst, und
wobei das Messen der tiefenspezifischen bioelektrischen Impedanz weiterhin das Messen einer ersten bioelektrischen Impedanz in einer ersten Tiefe in dem Messbereich des optischen Sensors und das Messen einer zweiten bioelektrischen Impedanz in einer zweiten Tiefe in dem Messbereich des optischen Sensors umfasst, und wobei die erste Tiefe und die zweite Tiefe voneinander verschieden sind; und
Bestimmen biometrischer Informationen des Objekts von Interesse auf der Grundlage des optischen Signals und der tiefenspezifischen bioelektrischen Impedanz unter Verwendung eines Modells zur Schätzung biometrischer Informationen, wobei das Modell zur Schätzung biometrischer Informationen eine Beziehung zwischen dem optischen Signal, der tiefenspezifischen bioelektrischen Impedanz und den biometrischen Informationen definiert.

12. Das Verfahren nach Patentanspruch 11, wobei das Messen der tiefenspezifischen bioelektrischen Impedanz umfasst:
Messen einer ersten bioelektrischen Impedanz in einer ersten Tiefe durch Anlegen eines ersten Stroms an das Objekt von Interesse über die erste Elektrode und die zweite Elektrode und Messen einer ersten Spannung, die zwischen der ersten Elektrode und der zweiten Elektrode anliegt; und
Messen einer zweiten bioelektrischen Impedanz in einer zweiten Tiefe durch Anlegen eines zweiten Stroms an das Objekt von Interesse über die dritte Elektrode und die vierte Elektrode und Messen einer zweiten Spannung, die zwischen der dritten Elektrode und der vierten Elektrode anliegt.

13. Das Verfahren nach Patentanspruch 11, wobei das Messen der tiefenspezifischen bioelektrischen Impedanz umfasst:
Messen einer ersten bioelektrischen Impedanz in einer ersten Tiefe durch Anlegen eines ersten Stroms an das Objekt von Interesse über die erste Elektrode und die zweite Elektrode und Messen einer ersten Spannung, die zwischen der ersten Elektrode und der zweiten Elektrode anliegt;
Verbinden der ersten Elektrode und der dritten Elektrode;
Verbinden der zweiten Elektrode und der vierten Elektrode; und
Messen einer zweiten bioelektrischen Impedanz in einer zweiten Tiefe durch Anlegen eines zweiten Stroms an das Objekt von Interesse über die erste Elektrode, die mit der dritten Elektrode verbunden ist, und die zweite Elektrode, die mit der vierten Elektrode verbunden ist, und Messen einer zweiten Spannung, die zwischen der ersten Elektrode, die mit der dritten Elektrode verbunden ist, und der zweiten Elektrode, die mit der vierten Elektrode verbunden ist, anliegt.

14. Das Verfahren nach Patentanspruch 11, wobei die biometrischen Informationen eine Körperfettmasse, eine fettfreie Masse, eine Muskelmasse, eine Skelettmuskelmasse, eine Grundumsatzrate, eine intrazelluläre Wassermasse, eine extrazelluläre Wassermasse, eine Körperwassermasse, eine anorganische Masse, eine viszerale Fettmasse, ein Blutflussvolumen, eine Kalorienaufnahme, ein Hämatokrit oder einen Blutzuckerspiegel umfassen.

## Revendications

1. Appareil (100, 1000, 1100) de mesure d'un signal biologique, comprenant :
un capteur optique (110, 1010) conçu pour émettre de la lumière dans une région de mesure vers un objet d'intérêt, et recevoir un signal optique reflété depuis l'objet d'intérêt ;
un capteur d'impédance bioélectrique (120, 1020) conçu pour mesurer une impédance bioélectrique spécifique à la profondeur dans la région de mesure du capteur optique, le capteur d'impédance bioélectrique comprenant :
au moins une première électrode et une seconde électrode qui sont espacées sous une distance prédéterminée à l'écart l'une de l'autre et qui sont disposées symétriquement par rapport au capteur optique ; et
un dispositif de mesure d'impédance conçu pour mesurer une première impédance bioélectrique à une première profondeur dans la région de mesure du capteur optique et mesurer une seconde impédance bioélectrique à une seconde profondeur dans la région de mesure du capteur optique, la première profondeur et la seconde profondeur étant différentes l'une de l'autre, et le capteur d'impédance bioélectrique étant conçu pour mesurer la première impédance bioélectrique à la première profondeur et la seconde impédance bioélectrique à la seconde profondeur en ajustant un espacement des électrodes, une surface des électrodes, ou une fréquence de mesure ; et
l'appareil comprenant en outre :
un processeur (1030) conçu pour déterminer des informations biométriques de l'objet d'intérêt sur la base du signal optique reçu et de l'impédance bioélectrique spécifique à la profondeur en utilisant un modèle d'estimation d'informations biométriques, le modèle d'estimation d'informations biométriques définissant une relation entre le signal optique, l'impédance bioélectrique spécifique à la profondeur et les informations biométriques.

2. Appareil selon la revendication 1, le capteur d'impédance bioélectrique comprenant :
une troisième électrode et une quatrième électrode qui sont espacées sous une seconde distance à l'écart l'une de l'autre et sont disposées symétriquement par rapport au capteur optique ; et
le dispositif de mesure d'impédance étant en outre conçu pour :
mesurer la première impédance bioélectrique à la première profondeur en utilisant la première électrode et la seconde électrode ; et
mesurer la seconde impédance bioélectrique à la seconde profondeur en utilisant la troisième électrode et la quatrième électrode.

3. Appareil selon la revendication 2, la première électrode et la seconde électrode étant interposées entre la troisième électrode et la quatrième électrode ; et/ou
des centres respectifs de la première électrode, la seconde électrode, la troisième électrode, la quatrième électrode, et le capteur optique formant une ligne droite.

4. Appareil selon la revendication 2, le capteur d'impédance bioélectrique comprenant en outre :
un dispositif de commande de mode conçu pour commander un mode de fonctionnement pour mesurer la première impédance bioélectrique à la première profondeur et la seconde impédance bioélectrique à la seconde profondeur.

5. Appareil selon la revendication 4, le dispositif de commande de mode étant conçu pour :
connecter le dispositif de mesure d'impédance à la première électrode et la seconde électrode dans un premier mode de fonctionnement ; et
connecter le dispositif de mesure d'impédance à la troisième électrode et la quatrième électrode dans un second mode de fonctionnement, préférablement,
le dispositif de mesure d'impédance comprenant :
une source de courant conçue pour :
appliquer un premier courant à l'objet d'intérêt par l'intermédiaire de la première électrode et de la seconde électrode dans le premier mode de fonctionnement ; et
appliquer un second courant à l'objet d'intérêt par l'intermédiaire de la troisième électrode et de la quatrième électrode dans le second mode de fonctionnement ; et
un voltmètre conçu pour :
mesurer une première tension appliquée entre la première électrode et la seconde électrode dans le premier mode de fonctionnement ; et
mesurer une seconde tension appliquée entre la troisième électrode et la quatrième électrode dans le second mode de fonctionnement.

6. Appareil selon la revendication 4 ou 5, le dispositif de commande de mode étant conçu pour :
déconnecter la première électrode et la troisième électrode dans un premier mode de fonctionnement,
déconnecter la seconde électrode et la quatrième électrode dans le premier mode de fonctionnement,
connecter la première électrode et la troisième électrode dans un second mode de fonctionnement, et
connecter la seconde électrode et la quatrième électrode dans le second mode de fonctionnement.

7. Appareil selon la revendication 6, le dispositif de mesure d'impédance comprenant :
une source de courant conçue pour :
appliquer un premier courant à l'objet d'intérêt par l'intermédiaire de la première électrode et de la seconde électrode dans le premier mode de fonctionnement, et
appliquer un second courant à l'objet d'intérêt par l'intermédiaire de la troisième électrode qui est connectée à la première électrode et par l'intermédiaire de la quatrième électrode qui est connectée à la seconde électrode dans le second mode de fonctionnement ; et
un voltmètre conçu pour :
mesurer une première tension appliquée entre la première électrode et la seconde électrode dans le premier mode de fonctionnement, et
mesurer une seconde tension appliquée entre la troisième électrode qui est connectée à la première électrode et la quatrième électrode qui est connectée à la seconde électrode dans le second mode de fonctionnement.

8. Appareil selon la revendication 1, le capteur d'impédance bioélectrique comprenant en outre :
un dispositif de commande de mode conçu pour commander un mode de fonctionnement pour mesurer l'impédance bioélectrique spécifique à la profondeur, préférablement,
le dispositif de commande de mode étant en outre conçu pour :
définir la fréquence de mesure à une première fréquence dans un premier mode de fonctionnement, et
définir la fréquence de mesure à une seconde fréquence dans un second mode de fonctionnement.

9. Appareil selon la revendication 8, le dispositif de mesure d'impédance comprenant :
une source de courant conçue pour :
appliquer un premier courant de la première fréquence à l'objet d'intérêt par l'intermédiaire de la première électrode et de la seconde électrode dans le premier mode de fonctionnement, et
appliquer un second courant de la seconde fréquence à l'objet d'intérêt par l'intermédiaire de la première électrode et de la seconde électrode dans le second mode de fonctionnement ; et
un voltmètre conçu pour mesurer une tension appliquée entre la première électrode et la seconde électrode.

10. Appareil selon l'une des revendications 1 à 9, les informations biométriques comprenant une masse grasse corporelle, une masse maigre, une masse musculaire, une masse de muscles squelettiques, un taux métabolique de base, une masse d'eau intracellulaire, une masse d'eau extracellulaire, une masse d'eau corporelle, une masse inorganique, une masse de graisse viscérale, un volume de flux sanguin, un apport en calories, l'hématocrite, ou un taux de glycémie.

11. Procédé de mesure d'un signal biologique qui est effectué par un appareil de mesure du signal biologique qui comprend un capteur optique, une première électrode et une seconde électrode qui sont espacées à une première distance à l'écart l'une de l'autre et sont disposées symétriquement par rapport au capteur optique, et une troisième électrode et une quatrième électrode qui sont espacées à une seconde distance à l'écart l'une de l'autre et sont disposées symétriquement par rapport au capteur optique, le procédé comprenant :
la mesure d'un signal optique en émettant de la lumière dans une région de mesure vers un objet d'intérêt et la réception d'une lumière reflétée depuis l'objet d'intérêt ;
la mesure d'une impédance bioélectrique spécifique à la profondeur dans la région de mesure du capteur optique en utilisant la première électrode, la seconde électrode, la troisième électrode, et la quatrième électrode, la mesure de l'impédance bioélectrique spécifique à la profondeur comprenant la mesure d'une impédance bioélectrique spécifique à la profondeur en ajustant un espacement des électrodes, une surface des électrodes, ou une fréquence de mesure, et
la mesure de l'impédance bioélectrique spécifique à la profondeur comprenant en outre la mesure d'une première impédance bioélectrique à une première profondeur dans la région de mesure du capteur optique et la mesure d'une seconde impédance bioélectrique à une seconde profondeur dans la région de mesure du capteur optique, et la première profondeur et la seconde profondeur étant différentes l'une de l'autre ; et
la détermination d'informations biométriques de l'objet d'intérêt sur la base du signal optique et de l'impédance bioélectrique spécifique à la profondeur en utilisant un modèle d'estimation d'informations biométriques, le modèle d'estimation d'informations biométriques définissant une relation entre le signal optique, l'impédance bioélectrique spécifique à la profondeur et les informations biométriques.

12. Procédé selon la revendication 11, la mesure de l'impédance bioélectrique spécifique à la profondeur comprenant :
la mesure d'une première impédance bioélectrique à une première profondeur en appliquant un premier courant à l'objet d'intérêt par l'intermédiaire de la première électrode et de la seconde électrode et la mesure d'une première tension appliquée entre la première électrode et la seconde électrode ; et
la mesure d'une seconde impédance bioélectrique à une seconde profondeur en appliquant un second courant à l'objet d'intérêt par l'intermédiaire de la troisième électrode et de la quatrième électrode et la mesure d'une seconde tension appliquée entre la troisième électrode et la quatrième électrode.

13. Procédé selon la revendication 11, la mesure de l'impédance bioélectrique spécifique à la profondeur comprenant :
la mesure d'une première impédance bioélectrique à une première profondeur en appliquant un premier courant à l'objet d'intérêt par l'intermédiaire de la première électrode et de la seconde électrode et la mesure d'une première tension appliquée entre la première électrode et la seconde électrode ;
la connexion de la première électrode et de la troisième électrode ;
la connexion de la seconde électrode et de la quatrième électrode ; et
la mesure d'une seconde impédance bioélectrique à une seconde profondeur en appliquant un second courant à l'objet d'intérêt par l'intermédiaire de la première électrode qui est connectée à la troisième électrode et la seconde électrode qui est connectée à la quatrième électrode et la mesure d'une seconde tension appliquée entre la première électrode qui est connectée à la troisième électrode et la seconde électrode qui est connectée à la quatrième électrode.

14. Procédé selon la revendication 11, les informations biométriques comprenant une masse grasse corporelle, une masse maigre, une masse musculaire, une masse de muscles squelettiques, un taux métabolique de base, une masse d'eau intracellulaire, une masse d'eau extracellulaire, une masse d'eau corporelle, une masse inorganique, une masse de graisse viscérale, un volume de flux sanguin, un apport en calories, l'hématocrite, ou un taux de glycémie.
